(19) Europäisches Patentamt

European Patent Office

Office européen des brevets

(11) **EP 1 225 444 A2**

(12) **EUROPÄISCHE PATENTANMELDUNG**

(43) Veröffentlichungstag:
   **24.07.2002  Patentblatt 2002/30**

(51) Int Cl.$^7$: **G01N 23/04**, **A61B 6/03**

(21) Anmeldenummer: **02100022.9**

(22) Anmeldetag: **17.01.2002**

(84) Benannte Vertragsstaaten:
   **AT BE CH CY DE DK ES FI FR GB GR IE IT LI LU MC NL PT SE TR**
   Benannte Erstreckungsstaaten:
   **AL LT LV MK RO SI**

(30) Priorität: **19.01.2001  DE 10102324**

(71) Anmelder:
   • **Philips Corporate Intellectual Property GmbH**
     **52066 Aachen (DE)**
   • **Koninklijke Philips Electronics N.V.**
     **5621 BA Eindhoven (NL)**

(72) Erfinder:
   • **Rasche, Volker Dr.,**
     **Philips Corp. Int. Prop. GmbH**
     **52066, Aachen (DE)**
   • **Klotz, Erhard P.A., Philips Corp. Int. Prop. GmbH**
     **52066, Aachen (DE)**
   • **Koppe, Reiner H. Dr.,**
     **Philips Corp. Int.Prop.GmbH**
     **Aachen, 52066 (DE)**

(74) Vertreter: **Volmer, Georg, Dipl.-Ing. et al**
   **Philips Corporate**
   **Intellectual Property GmbH**
   **Weisshausstr. 2**
   **52066 Aachen (DE)**

(54) **Röntgeneinrichtung für die Tomosynthese**

(57)   Die Erfindung betrifft eine Röntgeneinrichtung zur Erstellung von Schichtbildern eines Untersuchungsobjekts (4) mit einer Röntgenquelle (1), einem Röntgendetektor (2), einer Transporteinrichtung (3) zum Bewegen des Untersuchungsobjekts (4) während der Erfassung einer Serie von Röntgenprojektionsabbildungen des Untersuchungsobjekts (4) in einer parallel zur Abbildungsebene (A) liegenden Bewegungsebene (B) und mit einer Steuereinrichtung (5) zur Steuerung der Erfassung der Röntgenprojektionsabbildungen und der Transporteinrichtung (3), wobei aus den Röntgenprojektionsabbildungen mittels eines Tomosyntheseverfahrens Schichtbilder des Untersuchungsobjekts (4) erstellt werden. Um die Erstellung der Schichtbilder von großen Untersuchungsobjekten oder großer Bereiche davon in möglichst kurzer Zeit zu ermöglichen, ist erfindungsgemäß vorgesehen, dass die Röntgenquelle (1) und der Röntgendetektor (2) während der Erfassung der Röntgenprojektionsabbildungen statisch angeordnet sind und dass die Steuereinrichtung (5) derart ausgestaltet ist, dass die Geschwindigkeit der Bewegung des Untersuchungsobjekts (4) und die Zeitpunkte zur Erfassung der Röntgenprojektionsabbildungen derart gewählt werden, dass alle abzubildenden Punkte eines Untersuchungsbereichs des Untersuchungsobjekts (4) in mindestens 10, vorzugsweise mindestens 50, verschiedenen Röntgenprojektionsabbildungen abgebildet werden.

FIG.1

**Beschreibung**

Die Erfindung betrifft eine Röntgeneinrichtung zur Erstellung von Schichtbildern eines Untersuchungsobjekts mit einer Röntgenquelle, einem Röntgendetektor, einer Transporteinrichtung zum Bewegen des Untersuchungsobjekts während der Erfassung einer Serie von Röntgenprojektionsabbildungen des Untersuchungsobjekts in einer parallel zur Abbildungsebene liegenden Bewegungsebene und mit einer Steuereinrichtung zur Steuerung der Erfassung der Röntgenprojektionsabbildungen und der Transporteinrichtung, wobei aus den Röntgenprojektionsabbildungen mittels eines Tomosyntheseverfahrens Schichtbilder des Untersuchungsobjekts erstellt werden. Außerdem betrifft die Erfindung ein entsprechendes Verfahren zur Erstellung von Schichtbildem eines Untersuchungsobjekts.

Die Erstellung von Schichtbildern mittels der Tomosynthese ist seit langem bekannt. Dabei werden die Röntgenquelle und der Röntgendetektor in parallelen Ebenen in entgegengesetzter Richtung zur Erfassung von Röntgenprojektionsabbildungen eines zwischen diesen Ebenen angeordneten Untersuchungsobjekts bewegt. Diese Bewegung erfolgt dabei derart, dass sich die Projektionslinie zwischen Röntgenquelle und Röntgendetektor, also die Verbindungslinie zwischen dem Fokuspunkt der Röntgenquelle und dem Zentrum des Röntgendetektors, für jede Röntgenprojektionsabbildung im selben Punkt des Untersuchungsobjekts schneidet. Mittels einfacher Addition der Bildwerte, die zu einem Voxel einer abzubildenden Schicht des Untersuchungsobjekts in unterschiedlichen Röntgenprojektionsabbildungen gehören, können dann unterschiedliche Schichtbilder erstellt werden. Während der Erfassung der Röntgenprojektionsabbildungen wird das Untersuchungsobjekt selbst nicht bewegt.

Aus der DE 197 56 697 A1 ist eine Vorrichtung zur Stückgut-Röntgentomosynthese bekannt. Dabei wird ein Stückgut mittels einer Fördereinrichtung linear durch eine Röntgenanordnung bewegt, welche mindestens drei Röntgenabtasteinheiten aufweist, deren Strahlenfächer so aufgespannt sind, dass das Stückgut aus unterschiedlichen Winkeln gleichzeitig durchleuchtet wird.

Bei den bekannten Verfahren kann immer nur ein sehr begrenzter Ausschnitt des Untersuchungsobjekts untersucht werden. Um auch ein größeres Untersuchungsobjekt zu untersuchen und Schichtbilder zu erstellen, muss das bekannte Verfahren mehrmals in unterschiedlichen Bereichen des Untersuchungsobjekts durchgeführt werden, wonach gegebenenfalls die separat erstellten Schichtbilder kombiniert werden können. Es ist jedoch wünschenswert, bereits mit einer einzigen Durchführung des Verfahrens größere Bereiche des Untersuchungsobjekts untersuchen zu können und Schichtbilder eines größeren Bereichs in möglichst kurzer Zeit zu erstellen, beispielsweise Ganzkörper-Schichtbilder eines Patienten oder Schichtbilder großer Gepäckstücke bei der Gepäckuntersuchung.

Der Erfindung liegt deshalb die Aufgabe zugrunde, eine insoweit verbesserte Röntgeneinrichtung und ein verbessertes Verfahren anzugeben, mit dem auch Schichtbilder größerer Untersuchungsbereiche in kürzerer Zeit möglich sind.

Diese Aufgabe wird ausgehend von einer eingangs genannten Röntgeneinrichtung bzw. einem eingangs genannten Verfahren dadurch gelöst, dass die Röntgenquelle und der Röntgendetektor während der Erfassung der Röntgenprojektionsabbildungen statisch angeordnet sind und dass die Steuereinrichtung derart ausgestaltet ist, dass die Geschwindigkeit der Bewegung des Untersuchungsobjekts und die Zeitpunkte zur Erfassung der Röntgenprojektionsabbildungen derart gewählt werden, dass alle abzubildenden Punkte eines Untersuchungsbereichs des Untersuchungsobjekts in mindestens 10, vorzugsweise mindestens 50, verschiedenen Röntgenprojektionsabbildungen abgebildet werden.

Der Erfindung liegt dabei die Erkenntnis zugrunde, dass Vorteilhafterweise nicht die Röntgenquelle und der Röntgendetektor, sondern vielmehr das Untersuchungsobjekt, also der Patient oder das zu untersuchende Gepäckstück während der Erfassung der Röntgenprojektionsabbildungen bewegt wird Dabei werden die Zeitpunkte der Erfassung der Röntgenprojektionsabbildungen und die Geschwindigkeit der Bewegung des Untersuchungsobjekts derart für die - vorliegend vorzugsweise digitale - Tomosynthese optimiert, dass jeder Punkt des abzubildenden Untersuchungsbereichs des Untersuchungsobjekts in einer Mindestanzahl von Röntgenprojektionsabbildungen erscheint, wobei diese Mindestanzahl mindestens 10, vorzugsweise mindestens 50 oder noch besser 100 bis 200, betragen soll, um eine ausreichende Bildqualität zu erreichen. Die Anzahl ist dabei jedoch stark abhängig von der Art, Beschaffenheit und Größe des Untersuchungsobjekts. Dadurch wird es ermöglicht, Schichtbilder auch größerer Objekte nach einer einzigen Durchführung des erfindungsgemäßen Verfahrens zu erstellen, beispielsweise Ganzkörper-Schichtbilder eines Patienten, wie es beim Trauma-Scanning erforderlich ist. Außerdem können die erfindungsgemäße Röntgeneinrichtung und das erfindungsgemäße Verfahren auch zur Materialprüfung und zur Gepäckuntersuchung eingesetzt werden, wobei die Integration in eine bestehende Bearbeitungskette möglich ist.

Um den Tomowinkel, also den Öffnungswinkel des von der Röntgenquelle durch das Untersuchungsobjekt hindurch auf den Röntgendetektor treffenden Räntgenstrahlenbündels, zu optimieren, ist in einer Ausgestaltung vorgesehen, dass der Abstand zwischen Röntgenquelle und Röntgendetektor veränderbar ist. Insbesondere soll die Position der Röntgenquelle in einer zur Detektorebene, die der Abbildungsebene entspricht, senkrechten Richtung veränderbar sein, während der Röntgendetektor statisch angeordnet ist. Dadurch kann der Tomowinkel derart optimiert werden, dass die Bildqualität optimiert wird. Je größer der Tomowinkel ist, desto besser ist auch die Bildqualität. Während der Erfassung der Röntgenprojektionsabbildungen wird die Position der Röntgenquelle bevorzugt jedoch nicht verändert.

In einer weiteren bevorzugten Ausgestaltung ist vorgesehen, mindestens zwei Serien von Röntgenprojektionsabbildungen zu erfassen, wobei die Röntgenquelle jeweils unterschiedliche Positionen innehat. Bevorzugt ist die Röntgenquelle dabei jeweils asymmetrisch bezüglich des Detektors angeordnet, also beispielsweise zur Erfassung der ersten Serie gegenüber dem linken Randbereich des Detektors und zur Erfassung der zweiten Serie gegenüber dem rechten Randbereich des Detektors angeordnet. Mit dieser Ausgestaltung kann eine weitere Optimierung des Tomowinkels und somit der Bildqualität erreicht werden.

Eine noch weitere Optimierung des Tomowinkels kann mittels mindestens eines weiteren in einer Ausgestaltung der Erfindung vorgesehenen Röntgendetektors erfolgen, welcher neben dem ersten Röntgendetektor angeordnet ist.

Bevorzugt ist erfindungsgemäß vorgesehen, dass die Transportvorrichtung zur Erfassung der Röntgenprojektionsabbildungen mit unregelmäßiger Geschwindigkeit und in unterschiedlichen, wechselnden Richtungen bewegt wird. Es hat sich gezeigt, dass die Bildung von Artefakten in den Schichtbildern verringert oder vermieden werden kann, wenn das Untersuchungsobjekt nicht geradlinig und kontinuierlich mit derselben Geschwindigkeit zur Erfassung der Röntgenprojektionsabbildungen durch den Röntgenstrahl bewegt wird, sondern in einer Art "chaotischer" Bewegung bewegt wird, wobei die Geschwindigkeit und die Bewegungsrichtung ständig wechselt. Es kann sogar vorteilhaft sein, die Transportvorrichtung manuell und völlig unregelmäßig zu bewegen, da sich dann am wenigsten Abbildungsfehler und Artefakte zeigen.

Weitere vorteilhafte Ausgestaltungen ergeben sich aus den Unteransprüchen. Es sei an dieser Stelle auch darauf hingewiesen, dass das erfindungsgemäße Verfahren zur Erstellung von Schichtbildern in identischer oder ähnlicher Weise weitergebildet sein und Ausgestaltungen aufweisen kann, wie dies bezüglich der erfindungsgemäßen Röntgeneinrichtung und in den auf Anspruch 1 rückbezogenen Unteransprüchen angegeben und vorstehend erläutert ist.

Die Erfindung wird nachfolgend anhand der Zeichnungen näher erläutert. Es zeigen:

Figur 1    eine schematische Darstellung einer erfindungsgemäßen Röntgeneinrichtung und

Figur 2    eine weitere Ausgestaltung einer erfindungsgemäßen Röntgeneinrichtung.

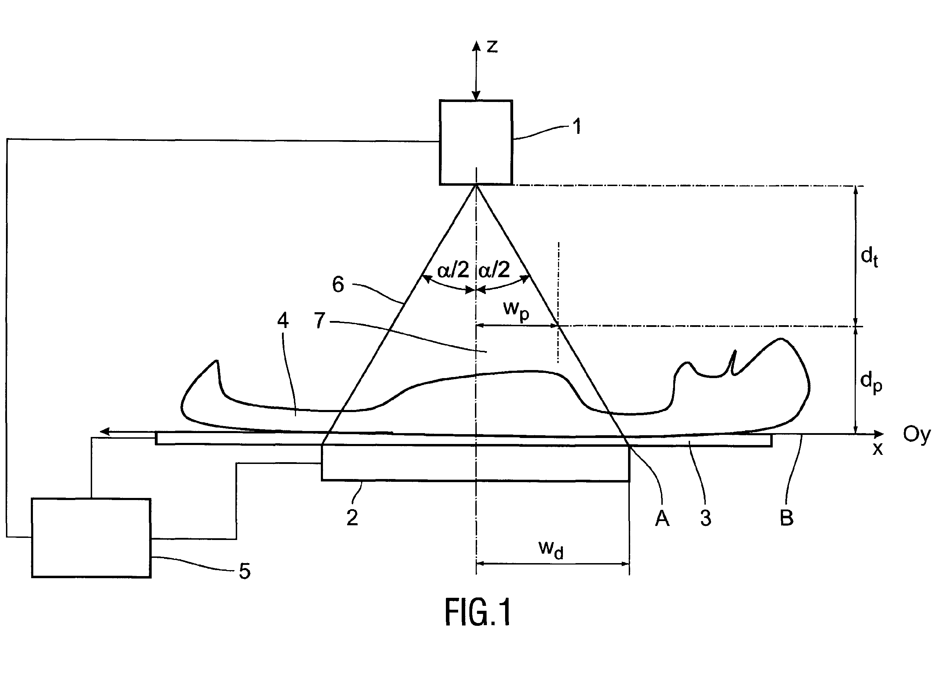

Die in Figur 1 gezeigte erfindungsgemäße Röntgeneinrichtung weist eine Röntgenquelle 1 und einem vorzugsweise flachen, zweidimensionalen Röntgendetektor 2 - dies kann auch ein Bildverstärker sein - auf, zwischen denen ein Patient 4 auf einer Transportvorrichtung 3, einem Patiententisch, angeordnet ist. Zur Erfassung von Röntgenprojektionsabbildungen wird die Transportvorrichtung 3 mit dem Patienten 4 in der Bewegungsebene B, also in x-und/oder y-Richtung bewegt, so dass das kegelstrahlförmige Röntgenstrahlenbündel 6 nacheinander unterschiedliche Bereiche des Patienten 4 durchtritt, die in der Abbildungsebene A auf dem Röntgendetektor 2 abgebildet werden. Die Bewegung des Patienten 4 wird dabei ebenso wie die Röntgenquelle 1 und der Röntgendetektor 2 von einer Steuereinrichtung 5 gesteuert. Zur Erfassung der Röntgenprojektionsabbildungen sind die Röntgenquelle 1 und der Röntgendetektor 2 statisch angeordnet. Allerdings ist die Position der Röntgenquelle 1 in z-Richtung veränderbar, um den Tomowinkel $\alpha$ zu optimieren.

Die resultierende Bildqualität der mittels der Tomosynthese erstellten Schichtbilder hängt wesentlich von dem Tomowinkel $\alpha$ und der Anzahl der Röntgenprojektionsabbildungen n, die für einen abzubildenden Punkt des Untersuchungsbereichs während der Datenerfassung erstellt wurden, ab. Der Tomowinkel $\alpha$ ergibt sich aus der Breite $w = 2w_d$ des Detektors 2 und dem Abstand $d = d_t + d_p$ zwischen dem Detektor 2 und der Röntgenquelle 1 zu:

$$\alpha = 2\tan^{-1}(w_d/d).$$

Die Anzahl der Projektionsabbildungen n resultiert aus der Zeit $t_b$, für die der abzubildende Punkt innerhalb des Röntgenstrahlenbündels liegt, und der Abbildungsrate $f_r$ des Detektors in:

$$n = f_r t_b.$$

Die Zeit $t_b$ resultiert aus der Geschwindigkeit $v_t$ der Transportvorrichtung und der Position z des Punktes entlang der Verbindungsachse 7 zwischen der Röntgenquelle und dem Röntgendetektor in:

$$t_b = (zw)/(dv_t).$$

Die maximale Geschwindigkeit $v_t^{max}$ hängt von der Abbildungsrate $f_r$ und der benötigten Anzahl von Röntgenprojektionsabbildungen n ab und ergibt sich zu:

$$v_t^{max} = (zwf_r)/(dn)$$

Dies soll nachstehend anhand eines Beispiels erläutert werden. Die maximale Höhe $d_p$ des Patienten sei $d_p = 50$ cm, z sei definiert durch $z = d - d_p$. Die Ab-

bildungsrate $f_r$ des Detektors sei $f_r$ = 25 Bilder pro Sekunde, und die Größe des Detektors sei 30 x 40 cm. Um eine ausreichende Abbildungsqualität zu erreichen, sollte jeder Punkt des Patienten bei den gegebenen Werten mindestens n = 100 mal abgebildet werden. In der nachfolgenden Tabelle sind die maximal benutzbaren Geschwindigkeiten für die Bewegung des Patienten und die resultierenden Tomowinkel für einen Detektor der oben beschriebenen Art aufgeführt.

| d [mm] | $W_p$[mm] | $\alpha$ [DEG] | $v_t$[mm/s] |
|--------|-----------|----------------|-------------|
| 1200   | 233       | 19             | 58          |
| 1000   | 200       | 23             | 50          |
| 800    | 150       | 28             | 37.5        |
| 600    | 67        | 37             | 17          |

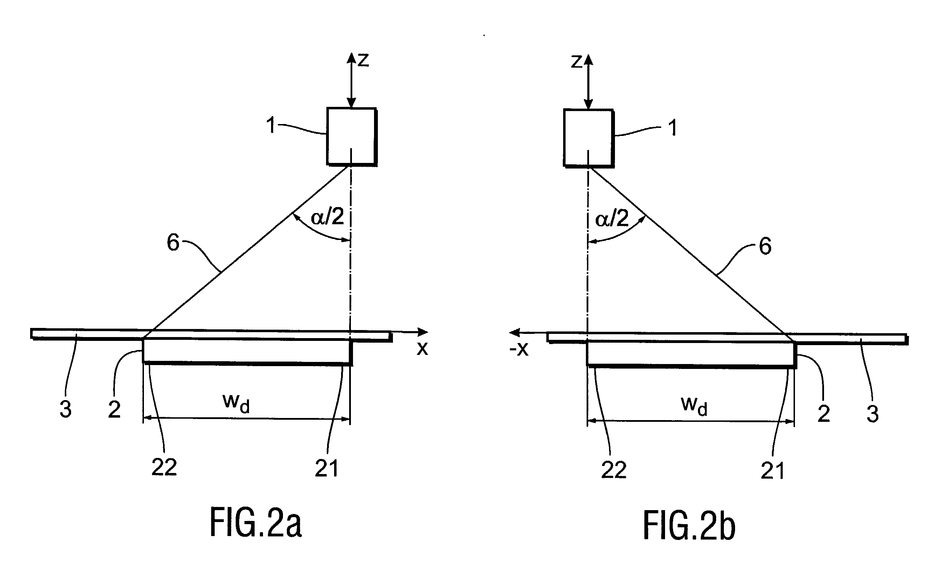

Da der erreichbare Tomowinkel nicht immer ausreichend groß ist, um hochqualitative Tomogramme bzw. Schichtbilder zu erstellen, können an dem beschriebenen Aufbau noch weitere Modifikationen vorgenommen werden. So kann beispielsweise, wie in Figur 2 gezeigt ist, eine asymmetrische Anordnung von Röntgenquelle und Röntgendetektor gewählt werden. Zur Erfassung einer ersten Serie von Röntgenprojektionsabbildungen ist die Röntgenquelle 1 dabei dem rechten Randbereich 21 des Detektors 2 gegenüberliegend angeordnet, wie in Figur 2a gezeigt ist, während zur Erfassung einer zweiten Serie von Röntgenprojektionsabbildungen die Röntgenquelle 1 dem linken Randbereich 22 des Detektors 2 gegenüberliegend angeordnet ist. Die Bewegungsrichtung der Transportvorrichtung 3 ist beim zweiten Durchlauf dabei invertiert. Mit einer solchen Anordnung kann eine Verdoppelung des Tomowinkels $\alpha$ erreicht werden. Grundsätzlich können auch mehr als zwei Serien von Röntgenprojektionsabbildungen erfasst werden, um den Tomowinkel weiter zu vergrößern.

Es kann auch vorgesehen sein, statt eines einzelnen Detektors ein oder mehrere weitere Detektoren zu verwenden, um die Detektorfläche zu vergrößern. Dies kann sowohl bei der in Figur 1 als auch bei der in Figur 2 gezeigten Ausgestaltung vorgesehen sein, wobei sich im letzteren Falle maximale Tomowinkel zwischen 90° (für $d_t$ = 100mm) und 62° (für $d_t$ = 500mm) für eine aus zwei Detektoren bestehende Ausgestaltung und von 107° (für $d_t$ = 100mm) bis zu 78° ($d_t$ = 500mm) für eine aus 4 Detektoren bestehende Ausgestaltung ergeben.

Um die Bildung von Artefakten zu vermeiden, kann statt einer motorischen Steuerung der Bewegung des Patienten während der Erfassung der Projektionsabbildungen auch vorgesehen sein, den Patiententisch mit dem Patienten manuell völlig unregelmäßig zu bewegen, das heißt insbesondere in ungeordneten Bewegungsrichtungen, wobei natürlich alle abzubildenden Punkte des Patienten ausreichend häufig vom Röntgenstrahl durchtreten werden müssen.

Bevorzugt sind weiterhin Mittel zur Messung der aktuellen Position der Transportvorrichtung des Patienten vorgesehen (nicht gezeigt in den Figuren). Diese sind vorzugsweise in der Transportvorrichtung selbst vorgesehen, beispielsweise als Potentiometer, deren Messwert proportional zur aktuellen Tischposition ist. Diese Positionsmessung wird zur Steuerung der Bewegung des Untersuchungsobjekts und zur Steuerung der Zeitpunkte zur Erfassung der Röntgenprojektionsabbildungen verwendet.

Die Erfindung kann nicht nur für medizinische Zwecke eingesetzt werden, sondern ist auch zur Materialprüfung oder zur Gepäckuntersuchung einetzbar, beispielsweise integriert in den Herstellungsprozess im Rahmen eines Fliessbandes oder eines Fördersystems.

## Patentansprüche

1. Röntgeneinrichtung zur Erstellung von Schichtbildern eines Untersuchungsobjekts (4) mit einer Röntgenquelle (1), einem Röntgendetektor (2), einer Transporteinrichtung (3) zum Bewegen des Untersuchungsobjekts (4) während der Erfassung einer Serie von Röntgenprojektionsabbildungen des Untersuchungsobjekts (4) in einer parallel zur Abbildungsebene (A) liegenden Bewegungsebene (B) und mit einer Steuereinrichtung (5) zur Steuerung der Erfassung der Röntgenprojektionsabbildungen und der Transporteinrichtung (3), wobei aus den Röntgenprojektionsabbildungen mittels eines Tomosyntheseverfahrens Schichtbilder des Untersuchungsobjekts (4) erstellt werden,
**dadurch gekennzeichnet,**
**dass** die Röntgenquelle (1) und der Röntgendetektor (2) während der Erfassung der Röntgenprojektionsabbildungen statisch angeordnet sind und dass die Steuereinrichtung (5) derart ausgestaltet ist, dass die Geschwindigkeit der Bewegung des Untersuchungsobjekts (4) und die Zeitpunkte zur Erfassung der Röntgenprojektionsabbildungen derart gewählt werden, dass alle abzubildenden Punkte eines Untersuchungsbereichs des Untersuchungsobjekts (4) in mindestens 10, vorzugsweise mindestens 50, verschiedenen Röntgenprojektionsabbildungen abgebildet werden.

2. Röntgeneinrichtung nach Anspruch 1,
**dadurch gekennzeichnet,**
**dass** die Geschwindigkeit der Bewegung des Untersuchungsobjekts (4) und die Zeitpunkte zur Erfassung der Röntgenprojektionsabbildungen derart eingestellt werden, dass alle abzubildenden Punkte eines Untersuchungsbereichs des Untersuchungsobjekts (4) in 100 bis 200 verschiedenen Röntgenprojektionsabbildungen abgebildet werden.

**3.** Röntgeneinrichtung nach Anspruch 1,
**dadurch gekennzeichnet,**
**dass** der Abstand zwischen Röntgenquelle (1) und Röntgendetektor (2) veränderbar ist.

**4.** Röntgeneinrichtung nach Anspruch 1,
**dadurch gekennzeichnet,**
**dass** zur Erstellung von Schichtbildern mindestens zwei Serien von Röntgenprojektionsabbildungen erfasst werden und dass die Röntgenquelle (1) zur Erfassung der Serien in unterschiedlichen Positionen angeordnet ist.

**5.** Röntgeneinrichtung nach Anspruch 4,
**dadurch gekennzeichnet,**
**dass** die Röntgenquelle (1) zur Erfassung der ersten Serie einem ersten Randbereich (21) des Röntgendetektors (2) gegenüberliegend und zur Erfassung der zweiten Serie einem zweiten Randbereich (22) des Röntgendetektors (2) gegenüberliegend angeordnet ist, wobei der erste Randbereich (21) dem zweiten Randbereich (22) gegenüberliegend angeordnet ist.

**6.** Röntgeneinrichtung nach Anspruch 1,
**dadurch gekennzeichnet,**
**dass** mindestens ein weiterer Röntgendetektor neben dem eisten Röntgendetektor (2) zur Erfassung von Röntgenprojektionsabbildungen angeordnet ist.

**7.** Röntgeneinrichtung nach Anspruch 1,
**dadurch gekennzeichnet,**
**dass** die Transportvorrichtung (3) manuell oder motorisch bewegbar ist und dass Mittel zur Messung der Tischposition vorgesehen sind.

**8.** Röntgeneinrichtung nach Anspruch 1,
**dadurch gekennzeichnet,**
**dass** die Transportvorrichtung (3) zur Erfassung der Röntgenprojektionsabbildungen mit unregelmäßiger Geschwindigkeit und in unterschiedlichen, wechselnden Richtungen, vorzugsweise manuell, bewegt wird.

**9.** Verfahren zur Erstellung von Schichtbildern eines Untersuchungsobjekts (4) mittels einer eine Röntgenquelle (1), einen Röntgendetektor (2), eine Transporteinrichtung (3) zum Bewegen des Untersuchungsobjekts (4) während der Erfassung einer Serie von Röntgenprojektionsabbildungen des Untersuchungsobjekts (4) in einer parallel zur Abbildungsebene (A) liegenden Bewegungsebene (B) und eine Steuereinrichtung (5) zur Steuerung der Erfassung der Röntgenprojektionsabbildungen und der Transporteinrichtung (3) aufweisenden Röntgeneinrichtung, wobei aus den Röntgenprojektionsabbildungen mittels eines Tomosyntheseverfahrens Schichtbilder des Untersuchungsobjekts (4) erstellt werden,
**dadurch gekennzeichnet,**
**dass** die Röntgenquelle (1) und der Röntgendetektor (2) während der Erfassung der Röntgenprojektionsabbildungen statisch angeordnet sind und dass die Geschwindigkeit der Bewegung des Untersuchungsobjekts (4) und die Zeitpunkte zur Erfassung der Röntgenprojektionsabbildungen derart gewählt werden, dass alle abzubildenden Punkte eines Untersuchungsbereichs des Untersuchungsobjekts (4) in mindestens 10, vorzugsweise mindestens 50, verschiedenen Röntgenprojektionsabbildungen abgebildet werden.

FIG.1

EP 1 225 444 A2

FIG.2a

FIG.2b

EP 1 225 444 A2